# EUROPEAN PATENT SPECIFICATION

(11) **EP 1 106 176 B1**
(45) Date of publication and mention of the grant of the patent: **18.06.2008**
(21) Application number: 00925584.5
(22) Date of filing: 10.05.2000
(51) Int. Cl.: A61K 31/343, A61K 31/5585, A61P 13/12

(54) **REMEDIES FOR RENAL FAILURE**
ARZNEIMITTEL GEGEN NIERENVERSAGEN
REMEDES A L'INSUFFISANCE RENALE

(30) Priority: 10.05.1999 JP 12913999
(43) Date of publication of application: 13.06.2001
(73) Proprietor: TORAY INDUSTRIES, INC., Tokyo 103-8666 (JP)
(72) Inventor: KURUMATANI, Hajimu, Kamakura-shi, Kanagawa 248-0034 (JP); SUZUKI, Motohiro, Kamakura-shi, Kanagawa 248-0031 (JP)
(74) Representative: Kador & Partner
(86) International application number: PCT/JP2000/002973
(87) International publication number: WO 2000/067748

(56) References cited:
- WO-A1-99/13880
- KUSHIRO MASAHIKO ET AL.: 'Therapeutic effects of prostacyclin analog on crescentic glomerulonephtitis of rat' KIDNEY INT. vol. 53, no. 3, 1998, pages 1314 - 1320, XP002930120
- UTSUNOMIYA Y. ET AL.: 'Attenuation of immune complex nephritis in NZB/W F1 mice by a prostacyclin analog' CLIN. EXP. IMMUNOL. vol. 99, no. 3, 1995, pages 454 - 460, XP002930121
- STEIR CHARLES T., JR. ET AL.: 'Beneficial action of beraprost sodium, a prostacyclin analog, in stroke-prone rats' J. CARDIOVASC. PHARMACOL. vol. 30, no. 3, 1997, pages 285 - 293, XP002930122

## Description

### Technical Field

The present invention relates to the use of beraprost, or a pharmacologically acceptable salt thereof for the manufacture of a medicament for chronic renal failure.

### Background Art

Prostaglandins (PGs) are a class of naturally occurring compounds with a wide variety of physiological activities, which have a common prostanoic acid skeleton. Naturally occurring PGs are classified into PGAs, PGBs, PGCs, PGDs, PGEs, PGFs, PGGs, PGHs, PGIs and PGJs according to the structural characteristics of the 5-membered ring in the skeleton. It is and also classified into subclasses 1, 2, 3 and so on according to the ansaturation and oxidation. Various synthetic analogues of these PGs are known. Among these, PGI₂, which is a typical PGI derivative, is called prostacycline (see Nature, vol. 268, p. 688, 1976). PGI₂ is known as a substance having potent platelet aggregation inhibiting activity and peripheral vasodilator activity. Japanese Examined Patent Application Publication Nos. 2-12226, 2-57548 and 1-53672 have described 4,8-inter-m-phenylene PGI₂ derivatives, in which the exo-enol ether moiety that is a structurally characteristic portion of PGI₂ is converted to an inter-m-phenylene moiety to substantially improve the instability of PGI₂. However, it has not yet recognized that such derivatives have therapeutic activities on renal failure.EP-A-1 016 408 discloses beraprost and its use for the treatment of nephritis, glomerulonephritis or diabetic nephropathy.

Renal failure is a condition characterized by decreased number of functional nephrons, resulting in reduced excretion of nitrogenous metabolic products and eventually causing the failure to maintain homeostasis in the biological environment. Specifically, this can be said to be a condition in which blood urea nitrogen (BUN) and creatinine levels are continuously increased. Renal failure is categorized into two primary types: acute renal failure in which the onset is abrupt and recovery may occur; and chronic renal failure which is slowly progressive but irreversible.

Acute renal failure is primarily categorized into the following two types: oliguric acute renal failure which is frequently complicated by water, electrolyte and acid-base imbalances and manifested by oliguria or anuria; and non-oliguric acute renal failure in which decreased urinary volume is not found.

Acute renal failure is also categorized into the following three types according to its cause: 1) pronephric acute renal failure in which reduction of renal blood flow occurs due to systemic hemodynamic changes such as prerenal dehydration and shock, causing reduced glomerular filtration rate; 2) renal acute renal failure which is induced by glomerular and tubular disorders such as acute tubular necrosis; and 3) postrenal acute renal failure which is caused by obstruction of the urinary tract, e.g., by a calculus. According to the clinical manifestations, it can also be categorized into oliguric, uretic and recovery stages. In the treatment of acute renal failure, it is important to track down its cause and sufficiently perform systemic control of the patient. Such treatment includes two major forms, conservative treatment and dialytic treatment. According to the conservative treatment, in the oliguric stage, excessive water drinking is avoided and the amount of protein intake is restricted, while simultaneously supplying a sufficient amount of calories. In the oliguric stage, or when heart failure has occurred, then sodium intake is restricted. In contrast, in the uretic stage, potassium intake is increased. Generally in the oliguric stage, calcium intake is restricted. In the case where BUN is 60 mg/dl or higher or rises by 30 mg/dl or more per day or hyperkalemia or heart failure is found, then it is recommended to perform an early frequent dialysis.

Chronic renal failure is a condition in which gradual reduction in renal functions occurs due to a chronically progressive renal disease, in which the reduced renal functions are manifested as the insufficiency of all functions for which the normal kidney is responsible. The causal diseases of chronic renal failure are all of the nephropathic diseases, including primary renal diseases, nephropathy in systemic diseases, congenital renal diseases, renal infections, nephropathy induced by any nephrotoxic substance and obstructive urinary diseases. As seen in the clinical background of patients to whom dialysis has been introduced for treatment of chronic renal failure, the primary causal diseases of chronic renal failure may include chronic glomerulonephritis, diabetic nephropathy, chronic pyelonephritis, nephrosclerosis and cystic kidney. Among these, chronic glomerulonephritis and diabetic nephropathy make up a large proportion. The proportion of diabetic nephropathy as the causal disease in the total cases; however, remarkably increases as the number of diabetic patients rapidly increases in recent years.

As stated above, renal failure may be caused by various diseases. However, all types of renal failure have particular common clinical manifestations regardless of their causal diseases, such as lung congestion and congestive heart failure associated with reduced urinary volume; neurological or mental complaints associated with advanced uremia; anemia caused by reduced production of erythropoietin in the kidney; electrolyte imbalance, such as hyponatremia and hyperkalemia; gastrointestinal complaints; defect of bone metabolism; and defect of carbohydrate metabolism.

For the treatment of chronic renal failure in the conservative stage, dietary therapy including a low-protein, high-calorie diet is basically employed. In this case, it is required to restrict sodium chloride intake and water intake and to use an antihypertensive agent to control the hypertention which may be a risk factor for exacerbation of renal failure. However, such dietary therapy and the treatment with an antihypertensive agent as mentioned above produce unsatisfactory effects. Therefore, the number of patients who inevitably have hemodialysis goes on increasing year by year due to the manifestation of uremic symptoms caused by the advanced disorders of renal functions. In patients with renal failure who have entered into dialysis, remarkable improvement in the rate of prolongation of life has been achieved due to the improved hemodialysis therapy in recent years. However, there still remain problems in that the patients are unavoidable to visit the hospital twice or three times a week, that defects of erythrocyte production or maturation may occur, that complications will follow which may caused by the accumulation of aluminum and β2-microglobulin in a body occurring after the long-term dialysis, and so on.

The object of the present invention is to provide a therapeutic agent for renal failure on which already-existing drugs or agents show unsatisfactory effects.

### Disclosure of Invention

The present invention provides the use of beraprost or a pharmacologically acceptable salt thereof for the manufacture of a medicament for chronic renal failure.

### Best Mode for Carrying Out the Invention

Beraprost sodium is represented by the following formula:

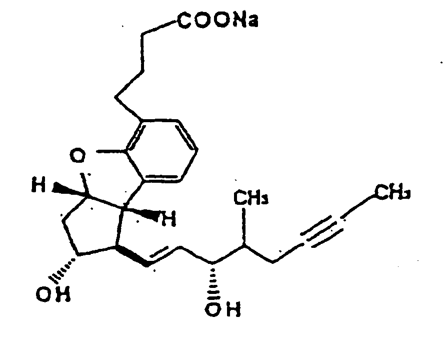

In the present invention, beraprost or a pharmacologically acceptable salt thereof can be administered at a dose of 0.001 to 1000 mg per adult subject once to three times a day.

Beraprost or a pharmacologically acceptable salt thereof may be administered as is. Alternatively, the therapeutic agent may be administered orally in the form of a solid preparation containing an additive or additives shown below.

The causal disease of the chronic renal failure to be treated in the present invention may include all of the nephropathic diseases, such as primary renal diseases, nephropathies in systemic diseases, congenital renal diseases, renal infections, nephropathies induced by any nephrotoxic substance and obstructive urinary diseases. Specific examples of the causal disease include, but are not limited to, chronic glomerulonephritis, diabetic nephropathy, chronic pyelonephritis, acute progressive nephritis, gestosis, cystic kidney, nephrosclerosis, malignant hypertension, nephropathies accompanied by various collagen diseases such as SLE, amyloid kidney, gouty kidney, disbolic renal failure, tuberculosis, renal calculosis, malignant tumor in the kidney and urinary tracts, obstructive urinary tract diseases, myeloma and renal hypoplasia.

Beraprost or a pharmacologically acceptable salt thereof is especially effective on chronic renal failure for which no effective therapy has currently been established and can delay the entrance into dialysis. Even when entered into dialysis, beraprost or a pharmacologically acceptable salt thereof may be effective for the preservation of functions of the remained kidney.

The additive may include excipients, such as starches, lactose, sucrose, glucose, mannitol, calcium carbonate and calcium sulfate; binders, such as starches, dextrin, gum arabic, gum tragacanth, methyl cellulose, gelatin, polyvinyl pyrrolidone and polyvinyl alcohol; disintegrating agents, such as starches, polyvinyl pyrrolidone and crystalline cellulose; lubricants, such as magnesium stearate and talk; coloring agents; flavoring agents; and so on.

Beraprost or a pharmacologically acceptable salt thereof invention may be administered in various dosage forms. Specifically, the dosage form may be any conventional one, such as tablets, dragees, powders, granules, troches, capsules, pills, syrup and spray.

Beraprost or a pharmacologically acceptable salt thereof may also be administered parentally in the form of a sterile solution. Sodium chloride, glucose or any other solute may be added to the solution, for example, in the amount sufficient to make the solution isotonic.

In addition to the dosage form for oral administration mentioned above, the therapeutic agent for renal failure of the present invention may be prepared in various dosage forms, such as various types of injections and suppositories for parenteral administration.

### [EXAMPLES]

The present invention will be described more in detail with reference to the following examples.

### EXAMPLE 1

### Effect of beraprost sodium on 5/6 nephrectomized rat model:

The effect of beraprost sodium on a 5/6 nephrectomized rat model, which has been widely used as a model animal for renal failure, was examined. Two-thirds of the left kidney was removed from each of 4-week-old male Wistar rats (Charles River Japan Inc.) with a razor, and all of the right kidney was then removed therefrom one week after. Three weeks after the initial surgery, blood was collected from each rat through the tail vein and serum creatinine and BUN levels were determined from the blood. At this point of time, the urine was also collected for 24 hours to determine the mass of proteins in the urine. The rats were allocated to one of the treatment groups by the stratifying continuous randomization method based on the mass of proteins in the urine and the body weight (n = 8 per group). There was observed little difference in the initial values of blood creatinine and BUN values among the rats. Three weeks after the initial surgery, beraprost sodium or captopril (SIGMA, i.e., a positive control) was orally administered to each rat twice a day everyday, beginning the day on which administration of a drug was started and continued through five weeks after the administration. The determination of the renal functions was performed both three weeks and five weeks after the initial administration of a drug. The photographic images of the renal tissue were observed only five weeks after the initial administration. In the sham surgery group ("sham") in which no drug was administered, increased BUN level (which is a measure of the progress of chronic renal failure) was observed three weeks after the initial administration, which indicated that chronic renal failure was advanced. Five weeks after the initial administration, more exacerbation of chronic renal failure was observed. In the beraprost sodium-administered group, significant prevention of increase in the urinary protein level and prevention of reduction in the creatinine clearance and increase in the BUN level were observed three weeks after the initial administration (Table 1). The same tendency was shown five weeks after the initial administration .(Table 2). As demonstrated by the observation of renal tissue images five weeks after the initial administration, the progress of the glomerular conditions was markedly prevented (Table 3). In the positive control group (i.e., the captopril-administered group), the similar ameliorative effects were shown. These results clearly demonstrate that beraprost sodium can improve the conditions of renal failure rats.

**Table 1: Effect of beraprost on chronic renal failure model rats (three weeks after the administration of a drug)**

| Drug | Body weight (g) | Urinary volume (ml/24hr) | Urinary protein excretion (mg/24hr/kgBW) | Creatinine clearance (µl/min/100gBW) | Blood urea nitrogen (µg/dl) |
|---|---|---|---|---|---|
| Sham | 229.4±6.1 | 32.3±3.7 | 3.6±0.6 | 430.9±15.3 | 14.9±1.1 |
| Control | 224.8±6.9 | 18.4±1.7** | 320.6±69.7** | 132.7±19.9** | 58.9±7.7* |
| Captopril (50 mg/kg) | 220.6±5.4 | 21.7±3.7 | 136.8±29.9 | 151.9±12.3. | 51.4±3.7 |
| TRK-100 (100µg/kg) | 215.5±5.3 | 20.1±2.8 | 125.3±20.8^{#} | 134.1±11.0 | 55.9±3.7 |
| TRK-100 (300µg/kg) | 221.0±6.3 | 20.4±3.7 | 154.4±53.0^{#} | 136.4±9.9 | 50.9±3.6 |

| | | | | | |
|---|---|---|---|---|---|
| Values represent mean ± s.e.m. from 8 rats. ** represents the control group having a significant difference from the sham group (p<0.01). # (p<0.05) and ## (p, 0.01) represent the drug-administered groups having a significant difference from the control group. | | | | | |

**Table 2: Effect of beraprost on chronic renal failure model rats (five weeks after the administration of a drug)**

| Drug | Body weight (g) | Urinary volume (ml/24hr) | Urinary protein excretion (mg/24hr/kgBW) | Creatinine clearance (µl/min/100gBW) | Blood urea nitrogen (µg/dl) |
|---|---|---|---|---|---|
| Sham | 238.4±7.1 | 24.7±4.1 | 7.8±0.7 | 365.6±20.1 | 16.9±1.1 |
| Control | 237.2±8.1 | 22.1±3.6 | 301.4±51.6** | 135.4±22.9** | 55.2±9.7* * |
| Captopril (50 mg/kg) | 236.4±5.7 | 21.9±3.2 | 255.5±76.6 | 161.1±17.3 | 47.8±5.2 |
| TRK-100 (100µg/kg) | 228.7±5.7 | 21.7±3.1 | 276.5±69.2 | 156.9±19.S | 45.5±3.3 |
| TRK-100 (300µg/kg) | 245.8±8.0 | 20.4±1.7 | 197.1±21.1 | 143.5±10.0 | 45.1±3.5 |

| | | | | | |
|---|---|---|---|---|---|
| Values represent mean ± s.e.m. from 7-8 rats. ** represents the control group having a significant difference from the sham group (p<0.01). | | | | | |

**Table 3**

| Region | Histopathological change | Sham | Control | Captopril (50 µg/kg) | BPS (100µg/kg) | BPS (300µg/kg) |
|---|---|---|---|---|---|---|
| Renal corpuscle | Glomerular hypertrophy | 0 | 8 | 1 | 3 | 2 |
| | Cellular filling in glomerulus | 0 | 7 | 4 | 7 | 4 |
| | Glomerulosclerosis | 0 | 3 | 2 | 2 | 1 |
| | Epidermal growth in Bowman's capsule | 0 | 5 | 2 | 1 | 1 |
| | Deposition of PAS + materials in | 0 | 3 | 1 | 2 | 1 |
| | Bowman's capsule | | | | | |
| | Coagulation of glomeruli in Bowman's | 0 | 5 | 2 | 2 | 1 |
| | capsule | | | | | |
| Uriniferous tuble | Basophilic degeneration of tubules | 0 | 8 | 6 | 8 | 8 |
| | Proliferation of proximal tubules | 0 | 8 | 8 | 8 | 8 |
| | Tubular dilation | 0 | 8 | 8 | 8 | 8 |
| | Proteinaceous urinary casts | 0 | 8 | 4 | 5 | 2 |
| Framework | Infiltration of mononuclear cells | 0 | 6 | 7 | 7 | 7 |

| | | | | | | |
|---|---|---|---|---|---|---|
| Each value represents the number of animals in which histopathological change was observed in 8 animals per group. | | | | | | |

### EXAMPLE 2

Renal failure rat models of which the primary disease was glomerulonephritis were used to examine the effect of different 4,8-inter-m-phenylene prostaglandin I₂ derivatives including beraprost sodium on the models. Eight-week-old male WKY rats (Charles River Japan Inc.) were administered intravenously with rabbit anti-rat glomerular basement membrane antiserum to induce glomerulonephritis. Two weeks after the induction, blood was collected from each rat through the tail vein to determine blood creatinine and BUN levels. The blood creatinine and BUN levels in the glomerulonephritis-induced rats were remarkably higher than those in the non-induced rats, which indicated.that the conditions of the rats progressed into renal failure. Four types of 4,8-inter-m-phenylene prostaglandin I₂ derivatives in total, including beraprost sodium, were individually administered subcutaneously to the rats from the back continuously with an osmotic pump (ALZET) for one week, beginning two weeks after induction of glomerulonephritis and continued through three weeks after the induction. The renal functions (i.e., blood creatinine and BUN levels) were determined one week after the initial administration of a drug. In a glomerulonephritis-induced group to which no drug was administered (i.e., a control group), blood creatinine and the BUN levels determined three weeks after the induction of glomerulonephritis were increased compared with those determined two weeks after the induction, which indicated that the conditions of the rats progressed into chronic renal failure. In a beraprost sodium-administered group, blood creatinine and BUN levels determined three weeks after the induction of glomerulonephritis were significantly decreased compared with those in the control group (Table 4). Beraprost sodium can improve the conditions of rats with renal failure.

**Table 4: Effect of beraprost sodium on renal failure rat models of which the primary disease is glomerulonephritis**

| | Blood creatinine (mg/dl) | | BUN (mg/dl) | |
|---|---|---|---|---|
| | 2 weeks after | 3 weeks after | 2 weeks after | 3 weeks after |
| | induction | induction | induction | induction |
| | (before | (1 week after | (before | (1 week after |
| | administration) | the initial administration) | administration) | the initial administration) |
| Normal group | 0.32 ± 0.02 | 0.30 ± 0.01 | 11 ± 1 | 18 ± 1 |
| Control group | 0.69 ± 0.09 * | 0.89 ± 0.12* | 33 ± 7 * | 40 ± 3 * |
| | | | | |
| Beraprost sodium-administered group | | | | |
| 60 µg/kg | 0.61 ± 0.07 * | 0.48 ± 0.04 # | 28 ± 3 * | 31 ± 2 # |
| 200 µg/kg | 0.62 ± 0.06 * | 0.49 ± 0.04 # | 30 ± 3 * | 25 ± 1 # |

| | | | | |
|---|---|---|---|---|
| *: p<0.05 vs. normal group (Student's t-test) #: p<0.05 vs. control group (Dunnett method) | | | | |

### EXAMPLE 3

Glomerulonephritis rat models were used to examine the effect of beraprost sodium on the rat models both in a stage where renal failure had not been found (i.e., the inflammatory stage) and a stage where BUN level was increased and the conditions were progressed into renal failure (i.e., the renal failure stage). Eight-week-old male WKY rats (Charles River Japan Inc.) were administered intravenously with rabbit anti-rat glomerular basement membrane antiserum to induce glomerulonephritis. Each of beraprost sodium, captopril (SIGMA) and prednisolone (Shionogi & Co., Ltd.) was orally administered to the rats everyday either for one week from day 1 through day 7 after induction of glomerulonephritis (i.e., during the inflammatory stage) or for two weeks beginning two weeks after the induction through four weeks after the induction (i.e., during the renal failure stage). The frequency of the administration was twice a day for beraprost sodium and captopril and once a day for prednisolone. After the initial administration of a drug, urinary total protein excretion (which is a measure of renal functions) was determined. In the inflammatory stage (i.e., from day 1 through day 7 after induction of glomerulonephritis), the urinary total protein excretion in a glomerulonephritis-induced group to which no drug was administered (i.e., a control group) was remarkably increased compared with that in a non-glomerulonephritis-induced group (i.e., a normal group) (Table 6). In a beraprost sodium-administered group, the increase in urinal total protein excretion was markedly prevented (Table 6). In both captopril- and prednisolone-administered groups, effective prevention of increase in urinal total protein excretion was observed (Table 6). On the other hand, in the renal failure stage (i.e., from two weeks after induction of glomerulonephritis through four weeks after the induction), the urinary total protein excretion in the glomerulonephritis-induced group without administration of a drug (i.e., the control group) was remarkably increased compared with that in a non-glomerulonephritis-induced group (i.e., a normal group); in contrast, in the beraprost sodium-administered group, the increase in urinal total protein excretion was markedly prevented (Table 6). In both the captopril- and prednisolone-administered groups, no effective prevention of increase in urinal total protein excretion was observed (Table 6).

These results indicate that both prednisolone and captopril are not effective on the rats in the renal failure stage although they are effective on the rats in the inflammatory stage. In contrast, it is clearly indicated that beraprost sodium can improve the conditions of the rats both in the inflammatory stage and the renal failure stage.

**Table 6: Effect of beraprost sodium, captopril and prednisolone on glomerulonephritis model rats in inflammatory and renal failure stages**

| Urinary total protein excretion (mg/24 hr) | | | | | |
|---|---|---|---|---|---|
| | Inflammatory stage | Renal failure stage | | | |
| | | Exp. 1 | | Exp. 2 | |
| | 7 days after induction (1 week after initial administration | 2 weeks after induction (before administration) | 3 weeks after induction (1 week after initial administration) | 2 weeks after induction (before administration) | 4 weeks after induction (2 week after initial administration) |
| Normal group | 19 ± 1 | 30 ± 6 | 20 ± 2 | 14 ± 1 | 17 ± 1 |
| Control group | 138 ± 9* | 370 ± 30* | 423 ± 55* | 324 ± 18* | 451 ± 77* |
| Beraproat-administered group | | | | | |
| 300 µg/kg | 17 ± 7# | 374 ± 21* | 300 ± 51# | 321 ± 23* | 295 ± 46# |
| Captopril-administered group | | | | | |
| 50 mg/kg | 75 ± 14# | N.D. | N.D. | 318 ± 22* | 504 ± 51 |
| 100 mg/kg | N.D. | N.D. | N.D. | 325 ± 6* | 517 ± 33 |
| Prednisolone-administered group | | | | | |
| 2 mg/kg | 38 ± 8# | 368 ± 20 | 456 ± 78 | N.D. | N.D. |

| | | | | | |
|---|---|---|---|---|---|
| *: p<0.05 vs. normal group (Student's t-test) #: p<0.05 vs. control group (Dunnett method) N.D.: Not determined. | | | | | |

### Industrial Applicability

The present invention provides the use according to claim 1.

## Claims

1. Use of beraprost or a pharmacologically acceptable salt thereof for the manufacture of a medicament for chronic renal failure.

2. Use according to claim 1, wherein the causal disease of the chronic renal failure is glomerulonephritis or diabetic nephropathy.

3. Use according to claim 1 or 2, wherein said chronic renal failure is defined by raise of serum creatinine level and/or blood urea nitrogen level.

4. Use according to claim 1 or 2, wherein said chronic renal failure is progressive chronic renal failure in which serum creatinine level and/or blood urea nitrogen level raise(s) with time.

## Patentansprüche

1. Verwendung von Beraprost oder eines pharmakologisch akzeptablen Salzes davon für die Herstellung eines Medikaments für chronisches Nierenversagen.

2. Verwendung nach Anspruch 1, worin die kausale Erkrankung des chronischen Nierenversagens eine Glomerulonephritis oder diabetische Nephropathie ist.

3. Verwendung nach Anspruch 1 oder 2, worin das besagte chronische Nierenversagen durch den Anstieg der Serumkreatininkonzentration und/oder der Blutharnstoffstickstoffkonzentration definiert ist.

4. Verwendung nach Anspruch 1 oder 2, worin das besagte chronische Nierenversagen ein progressives chronische Nierenversagen ist, in dem die Serumkreatininkonzentration und/oder die Blutharnstoffstickstoffkonzentration mit der Zeit ansteigt/ansteigen.

## Revendications

1. Utilisation de béraprost ou d'un sel pharmacologiquement acceptable de celui-ci pour la fabrication d'un médicament contre l'insuffisance rénale chronique.

2. Utilisation selon la revendication 1, dans laquelle la maladie causale de l'insuffisance rénale chronique est la glomérulonéphrite ou la néphropathie diabétique.

3. Utilisation selon la revendication 1 ou 2, dans laquelle l'insuffisance rénale chronique est définie par une augmentation du niveau de créatinine dans le sérum et/ou du niveau d'azote uréique dans le sang.

4. Utilisation selon la revendication 1 ou 2, dans laquelle l'insuffisance rénale chronique est l'insuffisance rénale chronique progressive dans laquelle le niveau de créatinine dans le sérum et/ou le niveau d'azote uréique dans le sang augmente(nt) avec le temps.
